(19) Europäisches Patentamt
European Patent Office
Office européen des brevets

(11) **EP 2 398 893 B1**

(12) **EUROPEAN PATENT SPECIFICATION**

(45) Date of publication and mention of the grant of the patent:
**17.12.2014 Bulletin 2014/51**

(51) Int Cl.:
***C12N 5/00*** *(2006.01)*

(21) Application number: **10704371.3**

(22) Date of filing: **19.02.2010**

(86) International application number:
**PCT/EP2010/052142**

(87) International publication number:
**WO 2010/094773 (26.08.2010 Gazette 2010/34)**

(54) **BREWING METHOD USING FUNGAL OR BACTERIAL PROTEASES**

BRAUVERFAHREN UNTER VERWENDUNG VON PROTEASEN AUS BAKTERIEN ODER SCHIMMELPILZEN

PROCÉDÉ DE BRASSAGE UTILISANT DES PROTEASES BACTERIENNES OU FONGIQUES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **19.02.2009 EP 09153182**

(43) Date of publication of application:
**28.12.2011 Bulletin 2011/52**

(73) Proprietor: **Novozymes A/S
2880 Bagsvaerd (DK)**

(72) Inventors:
• **KREISZ, Stefan
DK-2610 Roedovre (DK)**
• **HELDT-HANSEN, Hans Peter
DK-2830 Virum (DK)**
• **OESTERGAARD, Peter Rahbek
DK-2830 Virum (DK)**
• **FREDERIKSEN, Anne Mette
DK-2100 Copenhagen Oe (DK)**
• **BAEKGAARD, Lone
DK-2000 Frederiksberg (DK)**

(56) References cited:
WO-A-2004/011591   WO-A-2004/072221
WO-A-2007/101888   WO-A2-02/46381
DE-B1- 2 223 656   US-A- 3 795 745

• **Fergus G.Priest, Graham G.Stewart: "Handbook of Brewing" 24 February 2006 (2006-02-24), Marcel Dekker Inc , XP009133020 ISBN: 082472657Xpages 333-381, page 360 - page 362**
• **VERNET THIERRY ET AL: "Structural and functional roles of asparagine 175 in the cysteine protease papain" JOURNAL OF BIOLOGICAL CHEMISTRY, vol. 270, no. 28, 1995, pages 16645-16652, XP002580993 ISSN: 0021-9258**

**Description**

**FIELD OF THE INVENTION**

This application is related to a method of brewing wherein a thermostable protease is used for managing the colloidal stability of beverages. The proteases may be obtainable from Nocardiopsis, Bacillus, or Thermoascus. The protease is added either to the mash during lautering and/or to the wort after lautering but before wort boiling.

**BACKGROUND OF THE INVENTION**

Many beverages like beer, wine, juice etc develop precipitates during manufacture or upon storage. This phenomenon is described as haze formation. Haze formation is generally believed to be due to interaction of proteins and polyphenols present in the beverage. This interaction leads to the formation of insoluble or semi-soluble suspension of colloidal particles. Since haze formation generally resembles cloudiness produced by microbial contamination, it is generally preferred that the beverages, particularly beer, are very clear and transparent even upon long storage. Hence processes have been developed to reduce such haze formation. These processes target either the proteins or the polyphenols or both.

Silica gels, Bentonite, Poly Vinyl Poly Pyrrolidone (PVPP) etc have been used to adsorb proteins and polyphenols, decreasing haze formation and improving colloidal stability. However such materials, when used repeatedly result in diminishing returns and consequently lead to increased costs. Moreover, they also remove other desirable compounds from the beverage, which may affect its quality.

Enzymes, particularly proteases, are also used during fermentation to improve the colloidal stability of beverages, particularly beer. Traditionally, proteases like papain and bromelain have been used to reduce chill haze formation. However these proteases are non specific and have been shown to affect the foam stability of the beverage by hydrolysing the proteins responsible for foam formation. Moreover these also cause flavour changes in the beverage. Another approach has been by the use of specific proteases that hydrolyse only the hydrophilic haze forming proteins but not the hydrophobic foam forming proteins. For example, a prolyl specific endoprotease is known which specifically hydrolyses the haze forming proteins thus improving colloidal stability and retaining foam stability. Use of proteases during fermentation might retain their activity in the final beer which is generally undesirable. Inactivation by pasteurization can furthermore lead to a burned flavour in the beer.

There still exists a need for improved processes for colloidal stabilization of beverages, especially beer.

**SUMMARY OF THE INVENTION**

In one aspect, the invention relates to a method of brewing a beer comprising:

A method of brewing a beer comprising adding a thermostable protease to a wort after lautering but before wort boiling, wherein thermostable means that the protease activity is at least 70% of the reference activity when tested according to the following method: The protease is diluted to 1mg/ml in a an assay buffer comprising 100 mM Succinic acid, 100mM HEPES, 100 mM CHES, 100 mM CABS, 1mM $CaCl_2$, 150 mM KCL, 0.01% Triton X-100, pH adjusted to 5.5 with NaOH. The protease is then pre-incubated on i) ice, ii) 10 min at 70°C. The substrate for which the protease has activity is suspended in 0.01% Triton X-100. To start the reaction, 20 $\mu$l is added to the tube and incubated in an eppendorf thermomixer at 70°C, 1400 rpm for 15 min. The reaction is stopped by placing the tubes on ice. The samples are centrifuged cold at 14000 $g$ for 3 min, and $OD_{590}$ is measured on the supernatant. The $OD_{590}$ value from the blank samples is subtracted from the corresponding values of the protease-treated samples. The thermostability of the protease is determined by calculating the percentage activity of the samples pre-incubated at 70° compared to the samples incubated on ice (100%).

In one aspect, the protease is obtainable from Nocardiopsis, Thermoascus or Bacillus.

In one aspect, the invention relates to a method of brewing a beer comprising contacting a wort after lautering but before wort boiling with a protease which comprises an amino acid sequence corresponding to Seq ID No 1, 2, or 3 and variants thereof which have at least 70% identity to Seq ID No 1, 2, or 3.

In one aspect, the method leads to a reduction in haze in the beer product.

In one aspect, the invention relates to a method of brewing a beer comprising contacting a mash during lautering and/or a wort after lautering but before wort boiling with a thermostable protease whereby the colloidal stability is increased when compared to beer brewed in a conventional way.

In one aspect, the contacting is done at a temperature of at least 60 degrees centigrade (herein after referred to as °C). In another aspect, the temperature is at least 70°C. In yet another aspect, the temperature is at least 74°C, more preferably at least 75°C, more preferably at least 76°C, more preferably at least 77°C and most preferably at least 78°C.

In one aspect, the contacting is done for a period between 10 minutes (here in after referred to as min) and 5 hours, preferably, the contacting is between 20 min and 3 hours, more preferably between 30 min and 2 hours and most preferably between 30 min and 1 hour.

**BRIEF DESCRIPTION OF DRAWINGS**

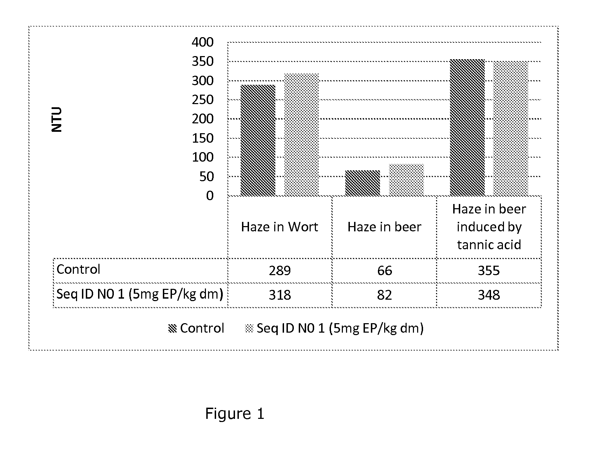

Fig. 1 shows the haze measurement in wort and beer received from a mash treated with a protease of Seq ID No 1 against untreated mash (control). The Y axis denotes the values in Nephelometric Turbidity Units (NTU).

**DETAILED DESCRIPTION OF THE INVENTION**

The process of beer-brewing is well known to the person skilled in the art. A conventional procedure may be outlined in the following way: The starting material is malted (i.e. dampened, germinated and subsequently dried) barley and/or unmalted adjuncts, called the grist. During the mashing, where the grist is grounded and mixed with water, heated and stirred, the carbohydrates are degraded to fermentable sugars by the aid of the enzymes naturally present in the malt. After mashing, it is necessary to separate the liquid extract (the wort) from the solids (spent grain particles and adjuncts) in order to get clear wort. This process is described as lautering. Prior to lautering, the mash temperature may be raised to about 75-78°C (165-173°F) (known as a mashout). Wort filtration is important because the solids are enriched in large amounts of protein, poorly modified starch, fatty material, silicates, and polyphenols (tannins) and proteins. The extract retained in the spent grain after collection of the first wort may also be washed out by adding hot water on top of the lauter cake. This process is called sparging. The hot water flows through the spent grain and dissolves the remaining extract. The diluted wort is called second wort and its extract decreases from the original gravity of the first wort down to 1-2 %. After addition of hops, the wort is boiled. Hereby numerous substances including several proteins are denatured and a precipitation of polyphenols will take place. After cooling and removal of precipitates, the finished beer wort (a) is aerated and yeast is added. After a main fermentation, lasting typically 5-10 days, most of the yeast is removed and the so called green beer (b) is stored at a low temperature, typically at 0 - 5°C for one to 12 weeks. During this period the remaining yeast will precipitate together with polyphenols. To remove the remaining excess polyphenols a filtration is performed. The fermented beer (c) may now be carbonized prior to bottling. Carbon dioxide not only contributes to the perceived "fullness" or "body" and as a flavor enhancer, it also acts as to enhance foaming potential and plays an important role in extending the shelf life of the product.

The term "beer" as used herein is intended to cover at least beer prepared from mashes prepared from unmalted cereals as well as all mashes prepared from malted cereals, and all mashes prepared from a mixture of malted and unmalted cereals. The term "beer" also covers beers prepared with adjuncts, and beers with all possible alcohol contents.

Without being bound by theory, it is believed that the interaction between proteins and polyphenols in beer stored at low temperatures or for a long time, lead to development of aggregates which are referred to as haze. Since the formation of haze affects the quality (also called as colloidal stability) of the beer, methods have been developed which prevents such haze formation. During the process of brewing, a majority of the protein-polyphenol complexes are precipitated out by cooling the liquid during beer maturation. Any remaining polyphenols and/or proteins are removed using PVPP, silica gel, bentonite etc.

According to this invention, colloidal stability of a beer is defined as the amount of warm cycles before the colloidal instability measured in EBC units becomes greater than 2. One warm cycle corresponds to approximately 25 days of shelf-life (MEBAK 2.15.2.1, Fociertmethode, Vorausbestimmung der chemisch-physikalischen stabilität, Methodensamlung der Mitteleuropäischen Brautechniche Analysekommission (MEBAK), Selbstverlag der MEBAK, Freising Weihenstephan).

Haze is also referred to as "cloudiness" or "turbidity" or "colloidal instability" in the art and hence can be used interchangeably.

Another method of reducing haze formation is by the use of proteases.

Proteases like papain, having broad spectrum activity, are used to cleave the proteins, possibly yielding lower and/or more soluble protein-polyphenol aggregates. However the use of these enzymes also affects the proteins involved in foam stability, thus affecting the quality of the final product.

Specific proteases like prolyl specific proteases or alanine specific proteases are also known which are hypothesised to act preferentially on haze active proteins and not on foam active proteins, thus preserving foam stability of the final product.

It is preferred that the final product is free from activity of external added enzymes. So enzymes, like the above proteases, are conventionally added to the mash (water + grist) during the process of mashing. Thus they are active during mashing and are inactivated or degraded during lautering and wort boiling.

The inventors have surprisingly found that addition of a protease to a mash during lautering and/or to a wort after the lautering step but before the wort boiling step leads to a good colloidal stabilization.

Surprisingly the inventors have found that proteases which either do not show any effect, or a direct negative effect on colloidal stabilisation when added in the beginning of mashing actually show very good increase in colloidal stability of beer when added during and/or after lautering. Lack of effect when the enzyme are added in mashing could have several reasons one being that the proteases are inhibited in the mash. The inventors have found that the negative effect of an protease added in mashing while being positive when added later in the process is related to the ability of the protease to release protein based matter into the wort when added early in the process which results in a negative impact on the colloidal stability even though the enzyme is capable of degrading the colloidal forming protein. The inventors have found that this can be avoided if the protease is added after lautering or sufficiently late in the process to limit the release of protein material into the wort, e.g. during lautering more specifically during sparging. Surprisingly the inventors have seen that the released amount of protein has to be kept to less than a 10% increase in the protein level measured by Kjehldal, preferably to less than 9 %, more preferably to less than 8%, more preferably to less than 7%, more preferably to less than 6%, and most preferably to less than 5%.

Surprisingly, the inventors have found that the amount of released protein in around MW of 10.000 KD (band 4-6) are kept at a relatively low level.

Surprisingly, it was also found that a protease which is not considered to be specific for action on haze active proteins and thereby not with low activity on foam active proteins not only can provide a good colloidal stabilization but also without any or only with a slight negative effect on the foam stability of the beer, when it is added to a mash during lautering and/or to a wort after lautering but before wort boiling.

Surprisingly, the inventors have found that proteases which are also thermostable provide good colloidal stabilization when added to a mash during lautering and/or to a wort after lautering but before wort boiling.

Surprisingly, the colloidal stabilization effect of the protease(s) applied to wort after lautering could be obtained within 2 hours which is much shorter than the several days when applied in fermentation.

The inventors have surprisingly found that thermostable proteases have very good colloidal stabilization activity when added to the mash during lautering and/or to the wort after lautering but before wort boiling.

The inventors have also found that use of a protease that substantially retains its activity during and/or after lautering in brewing surprisingly leads to increased colloidal stability of the beer.

Thus the invention in one aspect, relates to a method of brewing a beer comprising contacting a mash during lautering and/or a wort after lautering but before wort boiling with a protease obtainable from Nocardiopsis, Bacillus or Thermoascus.

The terms "mash", "lautering" and "wort" have the conventional meaning in the art.

Proteases are known in the art. They are polypeptides having protease activity and are also referred to as peptidases, proteinases, peptide hydrolases, or proteolytic enzymes. Proteases may be of the exo-type that hydrolyses peptides starting at either end thereof, or of the endo-type that act internally in polypeptide chains (endopeptidases). Endopeptidases show activity on N- and C-terminally blocked peptide substrates that are relevant for the specificity of the protease in question. The term "protease" is defined herein as an enzyme that hydrolyses peptide bonds. It includes any enzyme belonging to the EC 3.4 enzyme group (including each of the thirteen subclasses thereof). The EC number refers to Enzyme Nomenclature 1992 from NC-IUBMB, Academic Press, San Diego, Calif., including supplements 1 5 published in Eur. J. Biochem. 1994, 223,1 5; Eur. J. Biochem. 1995, 232, 1 6; Eur. J. Biochem. 1996, 237, 1 5; Eur. J. Biochem. 1997, 250, 1 6; and Eur. J. Biochem. 1999, 264, 610 650; respectively. The nomenclature is regularly supplemented and updated; see e.g. the World Wide Web (WWW) at www.chem.qmw.ac.uk/iubmb/enzyme/index.html.

Proteases are classified on the basis of their catalytic mechanism into the following groups: Serine proteases (S), Cysteine proteases (C), Aspartic proteases (A), Metallo proteases (M), and Unknown, or as yet unclassified, proteases (U), see Handbook of Proteolytic Enzymes, A. J. Barrett, N. D. Rawlings, J. F. Woessner (eds), Academic Press (1998), in particular the general introduction part.

Protease activity can be measured using any assay, in which a substrate is employed, that includes peptide bonds relevant for the specificity of the protease in question. Assay-pH and assay-temperature are likewise to be adapted to the protease in question. Examples of assay-pH-values are pH 2, 3, 4, 5, 6, 7, 8, 9, 10, 11, or 12. Examples of assay-temperatures are 30, 35, 37, 40, 45, 50, 55, 60, 65, 70, 80, 90, or 95 C. Examples of protease substrates include but not limited to casein, such as Azurine-Crosslinked Casein (AZCL-casein) and Protazyme AK.

There are no limitations on the origin of the protease of the invention and/or for use according to the invention. Thus, the term protease includes not only natural or wild-type proteases obtained from microorganisms of any genus, but also any mutants, variants, fragments etc. thereof exhibiting protease activity, as well as synthetic proteases, such as shuffled proteases, and consensus proteases. Such genetically engineered proteases can be prepared as is generally known in the art, e.g. by Site-directed Mutagenesis, by PCR (using a PCR fragment containing the desired mutation as one of the primers in the PCR reactions), or by Random Mutagenesis. The preparation of consensus proteins is described in e.g. EP 897985. Gene shuffling is generally described in e.g. WO 95/22625 and WO 96/00343. Recombination of protease genes can be made independently of the specific sequence of the parents by synthetic shuffling as described in Ness, J. E. et al, in Nature Biotechnology, Vol. 20 (12), pp. 1251 1255, 2002. Synthetic oligonucleotides degenerated in their DNA sequence to provide the possibility of all amino acids found in the set of parent proteases are designed and the genes assembled according to the reference. The shuffling can be carried out for the full length sequence or for only part of the sequence and then later combined with the rest of the gene to give a full length sequence.

In one aspect, the protease is obtainable from *Nocardiopsis.*

Nocardiposis has been described by Meyer in 1976 (Meyer, 1976, Int J Syst Bacteriol, 26, 487-493). *Nocardiopsis dassonvillei* is one of the species of this genus and is variously called as *Nocardiopsis dassonvillei subsp. dassonvillei, Nocardiopsis antarctica* or even *Streptomyces flavidofuscus.* Preferably, the protease is obtainable from the genus Nocardiopsis, more preferably *Nocardiopsis dassonvillei, Nocardiopsis alba,* or *Nocardiopsis antarctica,* more preferably *Nocardiopsis dassonvillei subsp. dassonvillei.*

In another aspect, the protease is obtainable from *Bacillus* or *Thermoascus.* Proteases obtainable from *Bacillus* are well known in the art. Proteases obtainable from *Thermoascus* are described in WO 03/048353.

The term "obtainable from" as used herein in connection with a given source shall mean that the polypeptide encoded by the nucleic acid sequence is produced by the source or by a recombinant cell (also called a host cell) in which the nucleic acid sequence from the source is present. In a preferred embodiment, the polypeptide is secreted extracellularly. Depending upon the host employed in a recombinant production procedure, the proteases of the present invention may be glycosylated or may be non-glycosylated. In addition, the proteases of the invention may also include an initial modified methionine residue, in some cases as a result of host-mediated processes.

The host cells may be a unicellular microorganism, e.g., a prokaryote, or a non-unicellular microorganism, e.g., a eukaryote.

Useful host cells are bacterial cells such as gram positive bacteria including, but not limited to, a Bacillus cell, or a Streptomyces cell, or cells of lactic acid bacteria; or gram negative bacteria such as E. coli and Pseudomonas sp. Lactic acid bacteria include, but are not limited to, species of the genera Lactococcus, Lactobacillus, Leuconostoc, Streptococcus, Pediococcus, and Enterococcus Other host cells can be fungal cells (including the phyla Ascomycota, Basidiomycota, Chytridiomycota, and Zygomycota (as defined by Hawksworth et al., In, Ainsworth and Bisby's Dictionary of The Fungi, 8th edition, 1995, CAB International, University Press, Cambridge, UK) as well as the Oomycota (as cited in Hawksworth et al., 1995, supra, page 171) and all mitosporic fungi (Hawksworth et al., 1995, supra).

In one aspect, the fungal host cell is a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in Biology and Activities of Yeast (Skinner, F. A., Passmore, S. M., and Davenport, R. R., eds, Soc. App. Bacteriol. Symposium Series No. 9, 1980). The yeast host cell may be a Candida, Hansenula, Kluyveromyces, Pichia, Saccharomyces, Schizosaccharomyces, or Yarrowia cell.

The fungal host cell may be a filamentous fungal cell. "Filamentous fungi" include all filamentous forms of the subdivision Eumycota and Oomycota (as defined by Hawksworth et al., 1995, supra). The filamentous fungi are characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as Saccharomyces cerevisiae is by budding of a unicellular thallus and carbon catabolism may be fermentative.

Examples of filamentous fungal host cells are cells of species of, but not limited to, Acremonium, Aspergillus, Fusarium, Humicola, Mucor, Myceliophthora, Neurospora, Penicillium, Thielavia, Tolypocladium, or Trichoderma.

In the production methods of the present invention, the cells are cultivated in a nutrient medium suitable for production of the protease using methods known in the art. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the protease is secreted into the nutrient medium, the same can be recovered directly from the medium. If the protease is not secreted, it can be recovered from cell lysates.

The resulting protease may be recovered by methods known in the art. For example, the protease may be recovered from the nutrient medium by conventional procedures including, but not limited to, centrifugation, filtration, extraction, spray-drying, evaporation, or precipitation.

The proteases of the present invention may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, e.g., Protein Purification, J.-C. Janson and Lars Ryden, editors, VCH Publishers, New York, 1989).

In one aspect, the protease comprises an amino acid sequence corresponding to Seq ID No 1 and variants there of which have at least 70% identity to Seq ID No 1.

In another aspect, the protease comprises and amino acid sequence corresponding to Seq ID No 2 or 3 and variants there of which have at least 70% identities to the corresponding parent sequence.

The term "variant" is intended to indicate a polypeptide which is derived from Seq ID No 1 or 2 or 3 and which has a protease activity. Typically, the variants differ from the parent protease (in this case, proteases having Seq ID NO 1 or 2 or 3 ) by one or more amino acid residues, which, for instance, may have been added or deleted from either or both of the N-terminal or C-terminal end of the protease, inserted or deleted at one or more sites within the amino acid sequence of the protease, or substituted for one or more amino acid residues within, or at either or both ends of the amino acid sequence of the parent protease. Variants can be both naturally occurring (for example, variants of polypeptides encoded by an allelic variant of a gene which is any of two or more alternative forms of a gene occupying the same chromosomal locus) or created artificially by many techniques known in the art (eg. PCR)

A protease according to the present invention comprises an amino acid sequence which has a degree of identity to the mature peptide part of SEQ ID NO: 1 or 2 or 3 , for example, at least about 70%. In particular embodiments, the degree of identity to SEQ ID NO: 1 or 2 or 3 or 4 is at least about 72%, 74%, 76%, 78%, 80%, 81%, 82%, 83%, 84%, 85%, 86%, 87%, 88%, 89%, 90%, 91%, 92%, 93%, 94%, 95%, 96%, 97%, 98%, or at least 99%. For the purposes of the present invention, the degree of identity between two amino acid sequences, is determined by the program "align" which is a Needleman-Wunsch alignment (i.e. a global alignment). The program is used for alignment of polypeptide, as well as nucleotide sequences. The default scoring matrix BLOSUM50 is used, with a penalty for the first residue of a gap being -12 and the penalties for further residues of a gap being -2.

"Align" is part of the FASTA package version v20u6 (see W. R. Pearson and D. J. Lipman (1988), "Improved Tools for Biological Sequence Analysis", PNAS 85:2444 2448, and W. R. Pearson (1990) "Rapid and Sensitive Sequence Comparison with FASTP and FASTA," Methods in Enzymology 183:63 98). FASTA protein alignments use the Smith-Waterman algorithm with no limitation on gap size (see "Smith-Waterman algorithm", T. F. Smith and M. S. Waterman (1981) J. Mol. Biol. 147:195 197).

The degree of identity between two amino acid sequences may also be determined by the Clustal method (Higgins, 1989, CABIOS 5: 151 153) using the LASERGENE.TM. MEGALIGN.TM. software (DNASTAR, Inc., Madison, Wis.) with an identity table and the following multiple alignment parameters: Gap penalty of 10, and gap length penalty of 10. Pairwise alignment parameters are Ktuple=1, gap penalty=3, windows=5, and diagonals=5.

In one aspect, the contacting of the protease with the wort is done after lautering.

The temperature at lautering is typically around 78 °C, it might therefore be needed to reduce the temperature after lautering to allow for the enzyme to work, reducing the temperature adds cost to the brewing process and it is therefore advantageous to reduce the temperature as little as possible and preferably not at all.

The temperature at which the enzyme is applied is preferably at least 60°C, more preferably at least 70°C, more preferably at least 72°C, more preferably at least 74°C, more preferably at least 75°C, more preferably at least 76°C, more preferably at least 77°C, and even more preferably at least 78°C.

In one aspect, the protease is a thermostable protease. Thermostability is a measure of an enzyme's ability to retain its activity at a higher temperature. For purposes of this invention the thermostability of a protease is measured using an assay on a protease substrate on which the protease is active, such as for example Protazyme AK ®, as described in example 3.

In one aspect the protease retains at least 70% activity, such as at least 75%, such as least 76%, such as at least 77%, such as at least 78%, such as at least 79%, such as at least 80%, such as at least 81%, such as at least 82%, such as at least 83%, such as at least 84%, such as at least 85%, such as at least 86%, such as at least 87%, such as at least 88%, such as at least 89%, such as at least 90%, such as at least 91%, such as at least 92%, such as at least 93%, such as at least 94%, such as at least 95%, such as at least 96%, such as at least 97%, such as at least 98%, such as at least 99% after incubation for 10 minutes at 70°C pH 5.5 using the buffer system described in example 3.

In one aspect, the thermostability can also be measured after incubation at 70°C for a longer time period such as for example 60 minutes using the assay described in example 3. In such cases, the protease retains at least 20% activity, such as at least 25%, such as at least 30%, such as at least 35%, such as at least 40%, such as at least 45%,such as at least 50%, such as at least 55%, such as at least 60%, such as at least 65%, such as at least 70%, such as at least 75%, such as at least 80%, such as at least 85%, such as at least 90%, such as at least 95%, such as at least 97%, such as at least 99% of its activity pH 5.5 using the buffer system described in ex 3.

In one aspect, the contacting is done for a period between 10 min and 5 hours, preferably between 20 min and 3 hours, more preferably between 30 min and 2 hours and most preferably between 30 min and 1 hour.

In one aspect, the method of the invention leads to a reduction in haze, when compared to a beer brewed in a conventional way.

To quantify the amount of haze in a beverage, a turbidimeter also called hazemeter is often used. In a turbidimeter the amount of light is measured that is scattered at a pre-described angle relative to the direction of the incident light beam. Turbidity measurements are very suitable for the measurement of haze formed as the result of protein-polyphenol interactions.

A "beer brewed in conventional way" is defined as a beer that has been made by a brewing method wherein the protease is added to the mash during the mashing stage.

In another aspect, the method of the invention leads to a decreased use of the processing aids used during brewing and storage to reduce the haze formation.

A "processing aid" is an agent that is used during brewing and/or storage to reduce the haze formation. The processing aids include but are not limited to silica gel, PVPP, bentonite etc.

In one aspect, the decrease is 100 percent, meaning no processing agents are used.

In another aspect, the beer is produced without filtration on silica and preferably without filtration on silica and PVPP.

In one aspect, the method of the invention leads to a beer that has foam stability either equal to or greater than a beer brewed in a conventional way.

Foam is formed by the carbon dioxide released due to release of pressure during dispensing of beer. The foam is made stable due to the presence of surface active agents like foam active proteins which collect on the bubble surface and form an elastic skin around the gas bubble. Foam stability of a beer is determined by measuring the time taken (in seconds) for the foam to collapse over a given distance using the NIBEM method (MEBAK 2.19.2). The method is known in the art (MEBAK 2.19.2., Schaumbestimmung nach NIBEM, Methodensamlung der Mitteleuropaischen Brautechniche Analysekommission (MEBAK), Selbstverlag der MEBAK, Freising Weihenstephan)

## EXAMPLES

**Example 1:** Role of protease of Seq ID No 1 in colloidal stabilization of beer when added either during mashing or to finished beer.

The objective was to investigate if the use of the above protease either in mash or in finished beer shows similar proteolytic activity and if there was any difference in colloidal stability.

The protease was obtained as described in US5312748. The proteolytic activity was determined by the well known Anson hemoglobin method, cfr. Journal of General Physiology, 22, 79-89 (1959). One Anson unit (AU) is the amount of proteolytic enzyme digesting hemoglobin at a pH value of 9.0 and a temperature of 25°C. during a reaction time of 10 minutes with such an initial velocity that per minute there is formed such an amount of split products which cannot be precipitated with trichloroacetic acid that these split products give the same color with phenol reagent as does one milli-equivalent of tyrosine.

In laboratory scale mashing trials with 100 % malt were executed. The mash was treated with the protease in an amount corresponding to 5 mg pure enzyme protein (EP)/kg malt protease (Seq ID No 1) following a mashing protocol shown in table 1. The control was also mashed according to the shown profile without adding the protease.

Table 1: mashing profile

| Time (min) | Temp (°C) |
| --- | --- |
| 0 | 52 |
| 30 | 52 |
| 40 | 62 |
| 70 | 62 |
| 80 | 72 |
| 110 | 72 |
| 116 | 78 |
| 131 | 20 |

The wort (12 °P) was fermented (12 °C, 5 days, Yeast strain W 35/70) and the haze of wort and beer was measured with a Turbidimeter (Turbidimeter Hach model 2100 AN). Furthermore the potential of forming haze in the final fermented beer was measured by forcing the haze formation with adding tannic acid according to a method published by Siebert 1997 (J. Am. Soc. Brew. Chem. 55(2):73-78, 1997).

The results are given in Figure 1.

From the result, it is evident that protease showed no influence on the turbidity in wort or beer, which is surprising. Even the addition of tannic acid showed no differences which indicates that the colloidal stability cannot be enhanced by adding tannins to the mash.

**Example 2:** Effect of proteases on colloidal stability and foam stability of beer when added during or after lautering but before wort boiling.

Wort was recovered from a typical 100% well modified German light pilsner malt industrial scale decoction mashing process according to the following profile: mashing-in at 62°C for 20 min, increase in temp to 65°C (1°C/min), rest at 65°C for 15 min, 20% of the mash was boiled for 20 min and mixed with the main mash kept at 65°C resulting in a mashing temperature of 72°C kept for 30 min, 20% of the mash was boiled and mixed with the main mash kept at 72°C resulting in a temperature of 78°C and lautering was initiated. Water was added during sparging resulting in malt to water ratio of 1:5.

The wort was diluted to Plato 12 and was split in three batches. Wort of batch A was not treated with enzyme (control), wort of batch B was incubated for 2 hours at 70°C with 5 mg enzyme protein/kg malt protease of Seq ID No 1 which corresponds to 0.019 AU/kg malt and wort of batch C was incubated for 2 hours at 60°C with 5 mg enzyme protein/kg malt of the proline-specific protease Brewers Clarex obtained from DSM (Het Overloon 1 6411 TE Heerlen (NL)) which corresponds to 2.7 g Brewers Clarex/100L beer. After termination of the enzyme incubations, the wort was boiled (inactivation of enzyme) for one hour with hops (8% $\alpha$-acids). The three wort batches went through the same brewing process in a pilot-scale process. Wort was pitched with 15x106 cells/mL of a typical bottom fermenting yeast type (W 34 70"). The fermentation was carried out at 12°C for 140 hours and the three beer batches were stabilized with PVPP (15 g/hL). Bottling of the beer in 0.5 L flasks was done manually.

The colloidal stability of the resulting beer samples was determined according to the procedure of MEBAK 2.15.2.1 (0°C/40°C/0°C). Formation of haze was measured in EBC (European Brewery Convention) units after up to 15 warm cycles. The shelf-life of a beer with emphasis on colloidal stability can be estimated by the number of warms cycles a beer can undergo until the haze measurement exceeds 2.0 EBC units [One warm cycle corresponds to approximately 25 days of shelf-life.

| Number of warm cycles | EBC units | | |
|---|---|---|---|
| | Batch A (control) | Batch B (Seq ID No 1) | Batch C (Brewers Clarex) |
| 0 | 0.6 | 0.3 | 0.3 |
| 1 | 0.8 | 0.4 | 0.3 |
| 2 | 0.8 | 0.4 | 0.4 |
| 3 | 1.1 | 0.4 | 0.3 |
| 4 | 1.4 | 0.4 | 0.4 |
| 5 | 1.7 | 0.4 | 0.4 |
| 6 | 1.9 | 0.4 | 0.4 |
| 7 | 2.2 | 0.4 | 0.4 |
| 8 | 2.6 | 0.4 | 0.6 |
| 9 | ND | 0.4 | 0.7 |
| 10 | ND | 0.3 | 0.9 |
| 11 | ND | 0.4 | 1.0 |
| 12 | ND | 0.4 | 1.3 |
| 13 | ND | 0.4 | 2.2 |
| 14 | ND | 0.4 | ND |
| 15 | ND | 0.4 | ND |

The data shows that the beer samples treated with Protease of Seq ID No 1 (Batch B) has the markedly best colloidal stability of all the samples (Batch A-C). Brewers Clarex (batch C) was furthermore performing better than the control sample (batch A).

The shelf life of the batches was estimated and is as below.

| | Batch A (control) | Batch B (Seq ID No 1) | Batch C (Brewers Clarex) |
|---|---|---|---|
| **Estimated shelf life (in days)** | 175 | ≥ 375 | 325 |

The data shows that the beer samples treated with protease of Seq ID No 1 (Batch B) has the highest shelf life of all the samples (Batch A-C). Brewers Clarex (batch C) also had better shelf life than the control sample (batch A).
The foam stability (NIBEM) was determined according to the procedure of MEBAK (2.19.2).

| | Batch A (control) | Batch B (Seq ID No 1) | Batch C (Brewers Clarex) |
|---|---|---|---|
| **NIBEM (in seconds)** | 246 | 261 | 267 |

The data shows that the samples treated with protease of Seq ID No 1 and Brewers Clarex (Batch B and C) can be described by having good foam stability properties to an extent which is better than in the control (batch A).

**Example 3:** Test for thermostability of proteases

The thermostability of the proteases of Seq ID No 1, Seq ID No 2 and Seq ID No 3 , and Neutrase® (commercially available from Novozymes A/S, Bagsvaerd, Denmark) was analysed using Protazyme AK® (Megazyme International Ireland Ltd, Wicklow, Ireland) as substrate. Alternatively, other substrates can also be used, if necessary, as long as the given protease has activity for the substrate.
The proteases of Seq ID No 1, 2, 3 and Neutrase were diluted to 1mg/ml in an assay buffer (100 mM Succinic acid, 100 mM HEPES, 100 mM CHES, 100 mM CABS, 1 mM $CaCl_2$, 150 mM KCl, 0.01% Triton X-100, pH adjusted to 5.5 with NaOH). The proteases were then pre-incubated on i) ice, ii) 10 min at 70°C. A blank sample (assay buffer without protease) was made and pre-incubated in the same way. Protazyme AK tablets were suspended in 0.01% Triton X-100, one tablet in 2 ml. For each reaction, 500 μl Protazyme solution was mixed with 500 μl assay buffer (described above) in an eppendorf tube and placed on ice. To start the reaction, 20 μl protease was added to the tube and incubated in an eppendorf thermomixer at 70°C, 1400 rpm for 15 min. The reaction was stopped by placing the tubes on ice. The samples were centrifuged cold at 14000 $g$ for 3 min, and $OD_{590}$ was measured on the supernatant. The $OD_{590}$ value for the blank samples was subtracted from the corresponding values of the protease treated samples. The thermostability of the proteases was determined by calculating the percentage activity of the samples pre-incubated at 70°C compared to the samples incubated on ice (100%). The results are given in the table below

**Table 1:** Thermostability of the proteases

| Protease | Incubation | % Activity[a] |
|---|---|---|
| Seq ID No. 1 | Ice | 100 |
| | 10 min at 70°C | 88 |
| Seq. ID No. 2 | Ice | 100 |
| | 10 min at 70°C | 71 |
| Seq ID. No. 3 | Ice | 100 |
| | 10 min at 70°C | 93 |
| Neutrase | Ice | 100 |
| | 10 min at 70°C | 0 |
| [a]Except from Neutrase, the values are an average of two measurements. | | |

From the table above, it is clear that proteases of Seq ID No. 1, 2, and 3 retained 88, 71 and 93 percent of their activity respectively at 70°C indicating that they are thermostable. However Neutrase did not retain any of its activity at 70°C indicating that it is not thermostable.

**Example 4:** Role of proteases of Seq ID No 1, 2, 3 and Neutrase in colloidal stabilization evaluated in a wort system.

Whether the proteases (Seq ID 1, 2, 3 and Neutrase) were capable of improving the colloidal stability of wort in comparison to a control was investigated as below:

Wort was recovered from a typical 100% well modified malt infusion mashing process with the following mashing profile: mashing-in at 54°C for 30 min, increase in temperature to 64°C (1°C/min), rest at 64°C for 60 min, increase in temperature to 78°C (1°C/min), rest at 78°C for 10 min followed by lautering (malt to water ratio in mashing 1:4). Water was added during sparging resulting in a malt to water ratio of 1:8.

The wort was incubated for 1 hour at 76°C according to the following specification:

   1) Control
   2) 8.0 mg EP protease of Seq ID No 1 EP/L wort
   3) 8.0 mg EP protease of Seq ID No 2 EP/L wort
   4) 8.0 mg EP protease of Seq ID No 3 EP/L wort
   5) 8.0 mg EP Neutrase /L wort

The wort was boiled for 30 min after the incubations in order to inactivate the enzyme.

The wort haze level, total nitrogen and free amino nitrogen (FAN) was analysed as below:

The wort haze level was determined by mixing 50 mL of filtered wort (filtered on a Sartorius Minisart-GF glass fibre filter) with 150 mL of water in the cuvette and the initial haze was measured ($Haze_{initial}$) on the Hazemeter (model: HZ 013, Lg-automatic ApS, DK) at a 90°C angle. The same sample was then put on the magnetic stirrer and 2x 3.75 mL of Brewtan C solution (200 mg Brewtan C (gallotannin, Ajinomoto OmniChem, Belgium)/L water) was added. The solution was incubated for 40 min and final haze was measured ($Haze_{final}$). Wort haze is calculated using the equation below:

$$\text{Wort haze} = Haze_{final} - Haze_{initial} \quad (Eq.\ 1)$$

Total Nitrogen (Kjeldahl) was analysed according to the Analytica EBC . Free amino nitrogen (FAN) was determined according to the procedure of MEBAK-II 2.9.4.11.

The result of the analysis is given in the table below:

**Table 2:** Role of proteases in colloidal stabilization evaluated in a wort system

|  | Control | Protease of Seq ID No 2 | Protease of Seq ID No 3 | Proteases of Seq ID No 1 | Neutrase |
|---|---|---|---|---|---|
| Wort haze | 9.3 ± 0.2 | 7.2 ± 0.0 | 2.7 ± 0.1 | 4.5 ± 0.0 | 10.6 ± 0.1 |
| FAN (mg/L) | 202.5 ± 0.7 | 206.5 ± 2.1 | 212.5 ± 0.7 | 207.0 ± 1.4 | 208.0 ± 0.0 |
| Total N (mg/Kg) | 941.5 ± 22.5 | 929.0 ± 17.0 | 959.5 ± 15.5 | 980.0 ± 0.0 | 980.0 ± 13.0 |

From the results above it is evident that proteases of Seq ID No. 1, 2, 3 positively affect wort haze .i.e. they result in decrease in wort haze when compared to a control (no enzyme treatment). Protease of Seq ID No. 3 showed the maximum effect followed by protease of Seq ID No 1 and 2 in that order. Neutrase negatively affects the wort haze.

Without being bound by theory, we believe that the negative effect of Neutrase is because of its lack of thermostability.

The FAN (Free Amino Nitrogen) did not seem to be greatly affected by the protease treatment. Protease of Seq ID No. 3 addition resulted in the biggest increase in wort FAN level.

Total nitrogen (N) measured by Kjeldahl method (Table 2). No major differences could be seen between the proteases treated samples and the control wort.

**Example 5:** Role of proteases of Seq ID No 1, 2 and 3 in colloidal stabilization evaluated in a beer system.

Whether the proteases (Seq ID 1, 2 and 3) were capable of improving the colloidal stability of beer in comparison to a control was investigated as below:

Wort was recovered from a typical 100% well modified German light pilsner malt industrial scale decoction mashing process according to the following profile: mashing-in at 62°C for 20 min, increase in temp to 65°C (1°C/min), rest at 65°C for 15 min, 20% of the mash was boiled for 20 min and mixed with the main mash kept at 65°C resulting in a mashing temperature of 72°C kept for 30 min, 20% of the mash was boiled and mixed with the main mash kept at 72°C resulting in a temperature of 78°C and lautering was initiated. Water was added during sparging resulting in malt to water ratio of 1:5.

The wort was diluted to Plato 12 and was split in 5 batches and 1-5 was incubated for 1 hour at 76°C with the following:

1) Protease of Seq ID No 3 (1.6 mg EP/L)
2) Protease of Seq ID No 1 (3.8 mg EP/L)
3) Protease of Seq ID No 2 (2.6 mg EP/L)
4) Control (no enzyme)
5) Control (fully stabilized beer: 15 g/L PVPP + 40 g/L silica gel)

After termination of the enzyme incubations, the wort was boiled (inactivation of enzyme) for one hour with hops (8% α-acids). The 5 wort batches went through the same brewing process in a pilot-scale process. Wort was pitched with $15 \times 10^6$ cells/mL of a typical bottom fermenting yeast type (W 34 70"). The fermentation was carried out at 12°C for 140 hours and the 5 beer batches were filtered. Bottling of the beer in 0.5 L flasks was done manually.

Total wort nitrogen and Total wort FAN was analysed as before. The results are given below.

**Table 3:** Total wort nitrogen (mg/100 mL, of 12°P).

| | Protease of Seq ID No.3 | Protease of Seq ID No.1 | Protease of Seq ID No.2 | Control | Control (fully stabillized after fermentation |
|---|---|---|---|---|---|
| Trial A | 105.6 | 104.7 | 105.5 | 104.0 | 104.4 |
| Trial B | 93.5 | 105.6 | 100.0 | 94.6 | 94.4 |

**Table 4:** Wort FAN (mq/100 mL, of 12°P)

| | Protease of Seq ID No.3 | Protease of Seq ID No.1 | Protease of Seq ID No.2 | Control | Contro (fully stabillized after fermentation |
|---|---|---|---|---|---|
| Trial A | 19.0 | 22.1 | 20.5 | 20.3 | 19.9 |
| Trial B | 19.0 | 18.5 | 17.8 | 18.7 | 18.2 |

From the results above, the wort Free Amino Nitrogen (FAN) and total nitrogen did not seem to be affected by the enzyme treatment.

The colloidal stability of the resulting beer samples was determined according to the procedure of MEBAK 2.15.2.1 (0°C/40°C/0°C). Formation of haze was measured in EBC units. The shelf-life of a beer with emphasis on colloidal stability can be estimated by the number of warms cycles a beer can undergo until the haze measurement exceeds 2.0 EBS units. One warm cycle corresponds to approximately 25 days of shelf-life. The results are given below:

**Table 5:** Colloidal stability results:

| # warm cycles | Protease of Seq ID No.3 | Protease of Seq ID No.1 | Protease of Seq ID No.2 | Control | Control (fully stabilized) |
|---|---|---|---|---|---|
| 0 | 0.459 | 0.424 | 0.375 | 0.801 | 0.649 |
| 1 | 1.43 | 0.764 | 1.03 | 2.01 | 1.46 |
| 2 | 1.47 | 0.748 | 1.09 | - | - |

(continued)

| # warm cycles | Protease of Seq ID No.3 | Protease of Seq ID No.1 | Protease of Seq ID No.2 | Control | Control (fully stabilized) |
|---|---|---|---|---|---|
| 3 | 1.58 | 0.782 | 1.17 | - | 1.68 |
| 4 | 1.58 | 0.788 | 1.21 | 2.35 | 1.50 |
| 5 | 1.62 | 0.805 | 1.23 | - | 1.72 |
| 6 | - | - | - | - | 1.78 |
| 7 | 1.80 | 0.849 | 1.36 | - | 1.86 |
| 8 | 1.61 | 0.810 | 1.34 | 2.15 | |
| 9 | 1.87 | 0.871 | 1.54 | 2.41 | |
| 10 | 1.86 | 0.870 | 1.70 | 2.61 | |
| 11 | 2.04 | 0.914 | 1.92 | 2.97 | |

From table 5, it demonstrates that treatment with Proteases of Seq ID No. 1, 2, 3 in particular after lautering at 76°C have a better colloidal stability than the control and fully stabilized control.

**Example 6:** Influence of Proteases on the wort quality with focus on the protein composition and haze formation in wort when added to different process steps.

The impact of proteases when applied at mashing, end of mashing and after lautering on the wort quality and protein composition was studied.

1.5 kg well modified malt was milled with a Heger MM40 2-Roller Mill (0.8 mm gap), and mashed in with 6 Liter of water (brewing quality). The mashing profile was 30 minutes at 52°C, 30 minutes at 62°C, 30 minutes at 72°C and 10 minutes at 78°C. The wort separation was done in a lauter tun. After collecting the first wort sparging was conducted using 2 times 3 Liter of 76°C brewing water. The wort was boiled 20 min in atmospheric conditions. 7 independent experiments was carried out and Protease of Seq ID No 1 was added according to the plan showed in Table 6

**Table 6:** Trial plan

| | Trial 1 | Trial 2 | Trial 3 | Trial 4 | Trial 5 | Trial 6 | Trial 7 |
|---|---|---|---|---|---|---|---|
| Point of addition | Mashing in | Mashing in | Mashing off | Mashing off | Wort | Wort | None |
| Enzyme dosage | 10 mg EP/Kg | 50 mg EP/Kg | 10 mg EP/Kg | 50 mg EP/Kg | 1.6 mg EP/L | 8.0 mg EP/L | None |

Wort samples were taken after boiling and analysed comprising the following analysis: Total Nitrogen (Kjeldahl), Free amino nitrogen (FAN), Haze (turbidity) after Brewtan C addition. The protein composition was also analysed by SDS-PAGE Wort Analysis (described in example 7 below). All methods were executed according to the Analytica EBC except the Brewtan C addition,which was done according to the procedure described in example 4 above. The results are given below:

**Table 7:** Results of the total N measurement using Kjehdahl method

| | Trial 1 | Trial 2 | Trial 3 | Trial 4 | Trial 5 | Trial 6 | Trial 7 |
|---|---|---|---|---|---|---|---|
| Point of addition | Mashing in | Mashing in | Mashing off | Mashing off | Wort | Wort | None |
| Enzyme dosage | 10 mg EP/Kg | 50 mg EP/Kg | 10 mg EP/Kg | 50 mg EP/Kg | 1.6 mg EP/L | 8.0 mg EP/L | None |
| Kjeldahl | 970 | 1287 | 996 | 1406 | 822 | 830 | 838 |
| PRIK | 16% | 54% | 19% | 68% | -2% | -1% | 0 |

The above results show that any contact of the protease with the mash increases the total N and therefore changes the wort and beer quality whereas contact of the protease with wort does not change the total Nitrogen content of the wort.

The Protein releasing index by Kjeldahl (PRIK) was also calculated.

The PRIK - or protein releasing index by Kjeldahl is hereby defined as the amount of released protein above the control without enzyme in percent of the control, i.e. PRIK = ((Kjeldahl protein of wort from sample with enzyme) - (Kjeldahl protein of control sample without enzyme))x100/(Kjeldahl protein of control sample without enzyme).

So our invention comprises a method, wherein the amount of protein in the wort, defined as the PRIK in accordance with Example 6, is increased less than 10%, preferably is increased less than 9%, more preferably is increased less than 8%, more preferably is increased less 7%, more preferably is increased less 6%, more preferably is increased less 5%, more preferably is increased less 4%, more preferably is increased less 3%, more preferably is increased less 2% and most preferably is increased less 1%.

**Table 8:** Results of the FAN measurement

|  | Trial 1 | Trial 2 | Trial 3 | Trial 4 | Trial 5 | Trial 6 | Trial 7 |
|---|---|---|---|---|---|---|---|
| Point of addition | Mashing in | Mashing in | Mashing off | Mashing off | Wort | Wort | None |
| Enzyme dosage | 10 mg EP/Kg | 50 mg EP/Kg | 10 mg EP/Kg | 50 mg EP/Kg | 1.6 mg EP/L | 8.0 mg EP/L | None |
| FAN (mg/L) | 198.9 | 217.7 | 212.2 | 257.4 | 189.7 | 192.8 | 174.2 |

As shown in the total N measurement above, the contact of mash with the protease increases the FAN level but not to the same extent like the total N. That indicates that proteins are solubilised when the protease is added to the mash but they are only partially hydrolysed to peptides or amino acids.

**Table 9:** Haze measurement of the boiled wort after treatment with Brewtan C

|  | Trial 1 | Trial 2 | Trial 3 | Trial 4 | Trial 5 | Trial 6 | Trial 7 |
|---|---|---|---|---|---|---|---|
| Point of addition | Mashing in | Mashing in | Mashing off | Mashing off | Wort | Wort | None |
| Enzyme dosage | 10 mg EP/Kg | 50 mg EP/Kg | 10 mg EP/Kg | 50 mg EP/Kg | 1.6 mg EP/L | 8.0 mg EP/L | None |
| Turbidity | 8.5 | 9.9 | 10.9 | 10.5 | 5.5 | 4.5 | 7.8 |
| GATA | -9% | -27% | -40% | -35% | 29% | 42% | - |

Where the GATA (Gallic Acid Turbidy Assay) is the reduction in turbidity relative to the control without protein as % of the control.

i.e GATA= [((Turbidity of control without protein)-(turbidity of sample))/(turbidity of control without protein)]X100

The results of the haze measurement indicate that the addition of the protease to the wort removes haze sensitive proteins whereas any contact of the protease with mash increases the total amount of Nitrogen and as a consequence this increases the potential of haze formation.

**Example 7:** Analysis of the wort proteins after treatment with proteases by SDS-PAGE Wort Analysis which is a Sodium dodecyl sulfate-polyacrylamide gel electrophoresis (SDS-PAGE) based method.

The wort obtained after protease treatment as mentioned in example 6 above was analysed using SDS-PAGE to understand the qualitative and quantitative changes in the protein profile, if any.

Samples were prepared for SDS-PAGE by adding 30 $\mu$l 4X Protein loading buffer containing dithiothreitol (DTT) (Fermentas, #R0891) to 90 $\mu$l sample resulting in 1X Protein loading buffer (0.0626 M Tris-HCl, pH 6.8, 2% SDS, 0.01% bromphenol blue, 10% glycerol, and 0.1 M DTT) followed by heating at 85°C for 3 min. 10 $\mu$l of the samples were added to a 4-20% Criterion Stain Free gel (Biorad) using a Tris/glycine/SDS running buffer (24 mM Tris, 0.2 M glycine, and 0.1% SDS). PageRuler™ Unstained Protein Ladder (Fermentas, #SM0661) was used as marker. The gel was run for 15 min at 100V and thereafter at 180V for approximately 1 hour until the front had reached the bottom of the gel. The proteins were visualized using Criterion Stain Free Imager (Biorad,California, USA).

From the SDS-PAGE, the different protein bands in the sample were quantified using the Image Lab Software for Criterion Stain Free system (Biorad).

6 protein bands, one following another, were identified for each sample using boiled wort as a reference.

Protein band 1 is the distinct band positioned at 40 kDa +/- 2 KDa. Protein bands 2, 3 and 4 are distinct bands positioned between 15 and 10 kDa, where band 2 is the largest (at 15 kDa +/- 2 KDa), band 4 is the smallest (just above 10 kDa) and band 3 is the distinct band in between band 2 and band 4. Protein band 5 and 6 -the next distinct bands with lower MW are found at 10 kDa and below, of which band 5 is the largest (approx. 10 kDa) and band 6 is the smallest (< 10 kDa).

The area of a protein band was marked with a box so it covered the whole band. In some cases, bands were so strong that they overlapped each other and here the bands were divided according to the sizes seen in the reference lane with boiled wort.

The intensity of the bands was calculated by the Image Lab Software Program. For each sample, the intensity of the individual bands was compared with the intensity of the same band in the reference (boiled wort) (Table 10). Furthermore, the quantity (%) of protein band 2 was calculated by dividing the intensity of the band with the sum of intensity of band 1 to 6 (Table 11).

**Table 10:** Quantity (%) of band 1, band 2, band 3 and band 4-6 in protease treated wort after boiling compared to the corresponding bands in boiled wort. Protease of Seq ID No 1 was added in two different concentrations; i) Low (L) (1.6 mg EP/L for wort and 10 mg EP/L for mash) and ii) high (H) (8 mg EP/L for wort and 50 mg EP/L for mash) at different steps in brewing (wort, mashing-in, and mashing off,).

| Sample | Band 1 | Band 2 | Band 3 | Band 4 | Band 5 | Band 6 | Band 4-6 | Total |
|---|---|---|---|---|---|---|---|---|
| Boiled Wort (no enzyme) | 100 | 100 | 100 | 100 | 100 | 100 | 100 | 100 |
| Wort (L) | 87.2 | 41.2 | 103.5 | 137.0 | 109.7 | 112.17 | 120.9 | 101.1 |
| Wort (H) | 84.1 | 14.0 | 71.8 | 88.7 | 178.1 | 118.7 | 119.6 | 92.2 |
| Mashing in (L) | 101.6 | 47.4 | 117.3 | 175.7 | 142.3 | 143.5 | 158.8 | 129.2 |
| Mashing in (H) | 123.7 | 27.3 | 116.9 | 207.4 | 436.6 | 402.4 | 307.2 | 215.1 |
| Mashing off (L) | 113.1 | 51.7 | 108.3 | 165.02 | 146.7 | 135.2 | 148.9 | 123.7 |
| Mashing off (H) | 122.6 | 40.6 | 106.8 | 220.0 | 277.3 | 277.8 | 242.9 | 178.7 |

The numbers are the average of two quantifications

**Table 11:** Quantity (%) of band 2 in wort after boiling compared to total protein (protein band 1 to 6) when Protease of Seq ID No. 1 was added in two different concentrations; i) Low (L) (1.6 mg EP/L for wort and 10 mg EP/L for mash) and ii) high (H) (8 mg EP/L for wort and 50 mg EP/L for mash) at different steps in brewing (wort, mashing-in, and mashing off).

| Sample | Band 2 |
|---|---|
| Sweet wort (no enzyme) | 15.7 |
| Boiled wort (no enzyme) | 15.5 |
| Wort - L | 6.3 |
| Wort - H | 2.4 |
| Mashing in (L) | 5.8 |
| Mashing in (H) | 2.0 |
| Mashing off (L) | 6.5 |
| Mashing off (H) | 3.7 |

*The numbers are the average of two quantifications*

Adding Protease of Seq ID No. 1 in a low and a high concentration to different steps in the brewing process changed the overall protein profile. By comparing the intensity of the individual protein bands from Protease of Seq ID No. 1 treated samples with boiled wort without enzyme; treatment with the protease to wort or mash significantly reduces protein band 2 (14.0-51.7% of band intensity in boiled wort). However, when the protease was added to mash, the protein bands 4-6 were significantly increased up to 307.2% compared to the bands in boiled wort (100%). Furthermore, addition of protease to wort resulted in a decrease in total protein amount (92.2% of total protein in boiled wort), whereas addition of the protease to mash resulted in increased amount of total protein (123.7-215.1% of total protein in boiled wort).

When the amount in percentage of protein band 2 was compared to the total amount of protein (band 1 to 6) for each sample, treating mash and wort with protease resulted in a significant decrease in band 2 (2.0-6.5% of total protein) compared to untreated sweet and boiled wort (15.5-15.7% of total protein) (Table 11).

Three other proteases from example 4, Protease of Seq ID No. 2, 3 and Neutrase, were tested for their protein degradation in sweet wort. The results are given below:

Table 12: Quantity (%) of band 1, band 2, band 3 and band 4-6 in wort treated with different proteases (8 mg EP/L) compared to the corresponding bands in boiled wort.

| Sample | Band 1 | Band 2 (CPRA) | Band 3 | Band 4-6 (CLRA) | Total |
|---|---|---|---|---|---|
| Boiled wort (no enzyme) | 100 | 100 | 100 | 100 | 100 |
| Protease Seq ID No. 3 | 90.3 | 13.8 | 28.8 | 152.2 | 107.9 |
| Protease Seq ID No. 2 | 56.4 | 48.8 | 114.8 | 141.7 | 113.7 |
| Protease Seq ID No. 1 | 93.6 | 26.4 | 120.4 | 183.5 | 140.8 |
| Neutrase | 120.2 | 116.6 | 158.8 | 152.7 | 144.1 |

*The numbers are the average of two quantifications*

So our invention also comprises a method, wherein the CPRA is below 80% and the CLRA is below 200%, preferably wherein the CPRA is below 70% and the CLRA is below 200%, more preferably wherein the CPRA is below 60% and the CLRA is below 200%, and most preferably wherein the CPRA is below 50% and the CLRA is below 200%.

Table 13: Quantity (%) of band 2 compared to total (protein band 1 to 6) in wort treated with Proteases (8 mg EP/L)

| Sample | Band 2 |
|---|---|
| Boiled wort (no enzyme) | 14.7 |
| Protease Seq ID No. 3 | 1.9 |
| Protease Seq ID No. 2 | 6.3 |
| Protease Seq ID No. 1 | 2.8 |
| Neutrase | 11.9 |

*The numbers are the average of two quantifications*

Both Protease of Seq ID No. 3 and 2 degraded band 2 down to 13.8-48.8% of the corresponding band in boiled wort (Table 11). Of the amount of total proteins, band 2 only accounted for 1.9-6.3% for Protease of Seq ID No. 1, 2 and 3 compared to 14.7% in boiled wort (Table 13). No major effect on protein band 2 was observed for Neutrase. (Table 12 and 13), which is probably due to less thermostability (T-opt (pH) = 50 (pH 9)).

## Claims

**1.** A method of brewing a beer comprising adding a thermostable protease to a wort after lautering but before wort boiling, wherein thermostable means that the protease activity is at least 70% of the reference activity when tested according to the following method:

The protease is diluted to 1mg/ml in a an assay buffer comprising 100 mM Succinic acid, 100mM HEPES, 100 mM CHES, 100 mM CABS, 1mM $CaCl_2$, 150 mM KCL, 0.01% Triton X-100, pH adjusted to 5.5 with NaOH. The protease is then pre-incubated on i) ice, ii) 10 min at 70°C. The substrate for which the protease has activity is suspended in 0.01% Triton X-100. To start the reaction, 20 $\mu$l is added to the tube and incubated in an eppendorf thermomixer at 70°C, 1400 rpm for 15 min. The reaction is stopped by placing the tubes on ice. The samples are centrifuged cold at 14000 g for 3 min, and $OD_{590}$ is measured on the supernatant. The $OD_{590}$ value from the blank samples is subtracted from the corresponding values of the protease-treated samples. The thermostability of the protease is determined by calculating the percentage activity of the samples pre-incubated at 70° compared to the reference activity, which is the protease activity of the samples incubated on ice.

2. The method according to claim 1, wherein the protease is obtainable from Nocardiopsis, Thermoascus or Bacillus.

3. The method according to claim 1, wherein the protease comprises an amino acid sequence corresponding to Seq ID No 1 or variants thereof which have at least 70% identity to Seq ID No 1.

4. The method according to claim 1, wherein the protease comprises an amino acid sequence corresponding to Seq ID No 2 or variants thereof which have at least 70% identity to Seq ID No 2.

5. The method according to claim 1, wherein the protease comprises an amino acid sequence corresponding to Seq ID No 3 or variants thereof which have at least 70% identity to Seq ID No 3.

6. The method according to claim 1, wherein the adding of the thermostable protease to the wort is done at a temperature of at least 60°C.

7. The method according to claim 1, wherein the adding of the thermostable protease to the wort is done at a temperature of at least 70°C.

8. The method according to claim 1, wherein the adding of the thermostable protease to the wort is done at a temperature of at least 75°C.

9. The method according to claim 1, wherein the adding of the thermostable protease to the wort is done for a period between 10 min and 5 hours.

10. The method according to claim 2, wherein the protease is obtainable from N. dassonvillei, Thermoascus arantiacus, or Bacillius licheniformis.

## Patentansprüche

1. Verfahren zum Brauen eines Bieres, umfassend das Hinzufügen einer thermostabilen Protease zu einer Würze nach dem Läutern aber vor dem Kochen der Würze, wobei thermostabil bedeutet, dass die Proteaseaktivität mindestens 70% der Referenzaktivität beträgt, wenn gemäß dem folgenden Verfahren getestet:

   Die Protease wird auf 1 mg/ml in einem Assaypuffer verdünnt, umfassend 100 mM Bernsteinsäure, 10 mM HEPES, 100 mM CHES, 100 mM CABS, 1 mM $CaCl_2$, 150 mM KCl, 0,01% Triton X-100, pH eingestellt auf 5,5 mit NaOH. Die Protease wird dann i) auf Eis, ii) 10 min bei 70°C vorinkubiert. Das Substrat, für das die Protease Aktivität aufweist, wird in 0,01% Triton X-100 suspendiert. Um die Reaktion zu starten werden 20 $\mu$l zu dem Röhrchen hinzugefügt und in einem Eppendorf Thermomixer bei 70°C, 1400 UpM für 15 min inkubiert. Die Reaktion wird durch Platzieren des Röhrchens auf Eis gestoppt. Die Proben werden kalt bei 14000 g für 3 min zentrifugiert, und $OD_{590}$ wird für den Überstand gemessen. Der $OD_{590}$-Wert der Blindproben wird von den entsprechenden Werten der Protease behandelten Proben subtrahiert. Die Thermostabilität der Protease wird bestimmt durch Berechnen der prozentualen Aktivität der bei 70 °C vorinkubierten Proben im Vergleich zu der Referenzaktivität, welches die Proteaseaktivität der auf Eis inkubierten Proben ist.

2. Verfahren nach Anspruch 1, wobei die Protease aus Nocardiopsis, Thermoascus oder Bacillus erhältlich ist.

3. Verfahren nach Anspruch 1, wobei die Protease eine Aminosäuresequenz umfasst, die SEQ ID NO: 1 entspricht, oder Varianten davon, die mindestens 70% Identität mit SEQ ID NO: 1 aufweisen.

**4.** Verfahren nach Anspruch 1, wobei die Protease eine Aminosäuresequenz umfasst, die SEQ ID NO: 2 entspricht, oder Varianten davon, die mindestens 70% Identität mit SEQ ID NO: 2 aufweisen.

**5.** Verfahren nach Anspruch 1, wobei die Protease eine Aminosäuresequenz umfasst, die SEQ ID NO: 3 entspricht, oder Varianten davon, die mindestens 70% Identität mit SEQ ID NO: 3 aufweisen.

**6.** Verfahren nach Anspruch 1, wobei das Hinzufügen der thermostabilen Protease zu der Würze bei einer Temperatur von mindestens 60 °C erfolgt.

**7.** Verfahren nach Anspruch 1, wobei das Hinzufügen der thermostabilen Protease zu der Würze bei einer Temperatur von mindestens 70 °C erfolgt.

**8.** Verfahren nach Anspruch 1, wobei das Hinzufügen der thermostabilen Protease zu der Würze bei einer Temperatur von mindestens 75 °C erfolgt.

**9.** Verfahren nach Anspruch 1, wobei das Hinzufügen der thermostabilen Protease zu der Würze für einen Zeitraum zwischen 10 min und 5 Stunden erfolgt.

**10.** Verfahren nach Anspruch 2, wobei die Protease aus N. dassonvillei, Thermoascus arantiacus oder Bacillus licheniformis erhältlich ist.

**Revendications**

**1.** Méthode de brassage d'une bière comprenant l'addition d'une protéase thermostable à un moût après la filtration mais avant la cuisson du moût, où thermostable signifie que l'activité protéase est au moins de 70 % de l'activité de référence selon la méthode suivante :

la protéase est diluée à 1 mg/ml dans un tampon de test comprenant 100 mM d'acide succinique, 100 mM de HEPES, 100 mM de CHES, 100 mM de CABS, 1 mM de $CaCl_2$, 150 mM de KCl, 0,01 % de Triton X-100, pH ajusté à 5,5 avec du NaOH. La protéase est ensuite préincubée sur i) de la glace, ii) 10 min à 70 °C. Le substrat pour lequel la protéase possède une activité est mis en suspension dans 0,01 % de Triton X-100. Pour démarrer la réaction, 20 μl sont ajoutés au tube et incubés dans un thermomixeur Eppendorf à 70 °C, 1400 tr/min pendant 15 min. La réaction est stoppée en déposant les tubes sur de la glace. Les échantillons sont centrifugés à froid à 14 000 g pendant 3 min, et la $DO_{590}$ est mesurée sur le surnageant. La valeur de $DO_{590}$ issue des échantillons témoins est soustraite des valeurs correspondantes des échantillons traités à la protéase. La thermostabilité de la protéase est déterminée en calculant le pourcentage d'activité des échantillons préincubés à 70 °C comparativement à l'activité de référence, qui est l'activité protéase des échantillons incubés sur de la glace.

**2.** Méthode selon la revendication 1, dans laquelle la protéase peut être obtenue à partir de Nocardiopsis, Thermoascus ou Bacillus.

**3.** Méthode selon la revendication 1, dans laquelle la protéase comprend une séquence d'acides aminés correspondant à SEQ ID NO : 1 ou ses variants qui présentent au moins 70 % d'identité avec SEQ ID NO : 1.

**4.** Méthode selon la revendication 1, dans laquelle la protéase comprend une séquence d'acides aminés correspondant à SEQ ID NO : 2 ou ses variants qui présentent au moins 70 % d'identité avec SEQ ID NO : 2.

**5.** Méthode selon la revendication 1, dans laquelle la protéase comprend une séquence d'acides aminés correspondant à SEQ ID NO : 3 ou ses variants qui présentent au moins 70 % d'identité avec SEQ ID NO : 3.

**6.** Méthode selon la revendication 1, dans laquelle l'addition de la protéase thermostable au moût est réalisée à une température d'au moins 60 °C.

**7.** Méthode selon la revendication 1, dans laquelle l'addition de la protéase thermostable au moût est réalisée à une température d'au moins 70 °C.

**8.** Méthode selon la revendication 1, dans laquelle l'addition de la protéase thermostable au moût est réalisée à une température d'au moins 75 °C.

9.  Méthode selon la revendication 1, dans laquelle l'addition de la protéase thermostable au moût est réalisée pendant une période comprise entre 10 min et 5 heures.

10. Méthode selon la revendication 2, dans laquelle la protéase peut être obtenue à partir de N. dassonvillei, Thermoascus arantiacus, ou Bacillus licheniformis.

| | Haze in Wort | Haze in beer | Haze in beer induced by tannic acid |
|---|---|---|---|
| Control | 289 | 66 | 355 |
| Seq ID N0 1 (5mg EP/kg dm) | 318 | 82 | 348 |

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 897985 A
- WO 9522625 A
- WO 9600343 A
- WO 03048353 A
- US 5312748 A

### Non-patent literature cited in the description

- Enzyme Nomenclature 1992 from NC-IUBMB. Academic Press, 1992
- *Eur. J. Biochem.,* 1994, vol. 223, 1-5
- *Eur. J. Biochem.,* 1995, vol. 232, 1-6
- *Eur. J. Biochem.,* 1996, vol. 237, 1-5
- *Eur. J. Biochem.,* 1997, vol. 250, 1-6
- *Eur. J. Biochem.,* 1999, vol. 264, 610-650
- Handbook of Proteolytic Enzymes. Academic Press, 1998
- **NESS, J. E. et al.** *Nature Biotechnology,* 2002, vol. 20 (12), 1251-1255
- **MEYER.** *Int J Syst Bacteriol,* 1976, vol. 26, 487-493
- **HAWKSWORTH et al.** Ainsworth and Bisby's Dictionary of The Fungi. University Press, 1995
- Soc. App. Bacteriol. Symposium Series No. 9. 1980
- Protein Purification. VCH Publishers, 1989
- **W. R. PEARSON ; D. J. LIPMAN.** Improved Tools for Biological Sequence Analysis. *PNAS,* 1988, vol. 85, 2444-2448
- **W. R. PEARSON.** Rapid and Sensitive Sequence Comparison with FASTP and FASTA. *Methods in Enzymology,* 1990, vol. 183, 63-98
- **T. F. SMITH ; M. S. WATERMAN.** Smith-Waterman algorithm. *J. Mol. Biol.,* 1981, vol. 147, 195-197
- **HIGGINS.** *CABIOS,* 1989, vol. 5, 151-153
- *Journal of General Physiology,* 1959, vol. 22, 79-89
- **SIEBERT.** *J. Am. Soc. Brew. Chem.,* 1997, vol. 55 (2), 73-78

SEQUENCE LISTING

<110> Novozymes A/S

<120> A brewing method

<130> 11567.204-WO

<160> 3

<170> PatentIn version 3.5

<210> 1
<211> 188
<212> PRT
<213> Nocardiopsis sp.

<220>
<221> mat_peptide
<222> (1) .. (188)

<400> 1

```
Ala Asp Ile Ile Gly Gly Leu Ala Tyr Thr Met Gly Gly Arg Cys Ser
1               5                   10                  15

Val Gly Phe Ala Ala Thr Asn Ala Ala Gly Gln Pro Gly Phe Val Thr
            20                  25                  30

Ala Gly His Cys Gly Arg Val Gly Thr Gln Val Thr Ile Gly Asn Gly
        35                  40                  45

Arg Gly Val Phe Glu Gln Ser Val Phe Pro Gly Asn Asp Ala Ala Phe
    50                  55                  60

Val Arg Gly Thr Ser Asn Phe Thr Leu Thr Asn Leu Val Ser Arg Tyr
65                  70                  75                  80

Asn Thr Gly Gly Tyr Ala Thr Val Ala Gly His Asn Gln Ala Pro Ile
                85                  90                  95

Gly Ser Ser Val Cys Arg Ser Gly Ser Thr Thr Gly Trp His Cys Gly
                100                 105                 110

Thr Ile Gln Ala Arg Gly Gln Ser Val Ser Tyr Pro Glu Gly Thr Val
        115                 120                 125

Thr Asn Met Thr Arg Thr Thr Val Cys Ala Glu Pro Gly Asp Ser Gly
        130                 135                 140
```

```
Gly Ser Tyr Ile Ser Gly Thr Gln Ala Gln Gly Val Thr Ser Gly Gly
145             150             155                 160


Ser Gly Asn Cys Arg Thr Gly Gly Thr Thr Phe Tyr Gln Glu Val Thr
                165             170                 175


Pro Met Val Asn Ser Trp Gly Val Arg Leu Arg Thr
            180             185
```

<210> 2
<211> 274
<212> PRT
<213> Bacillus licheniformis

<220>
<221> mat_peptide
<222> (1) .. (274)

<400> 2

```
Ala Gln Thr Val Pro Tyr Gly Ile Pro Leu Ile Lys Ala Asp Lys Val
1               5               10              15

Gln Ala Gln Gly Phe Lys Gly Ala Asn Val Lys Val Ala Val Leu Asp
            20              25              30

Thr Gly Ile Gln Ala Ser His Pro Asp Leu Asn Val Val Gly Gly Ala
        35              40              45

Ser Phe Val Ala Gly Glu Ala Tyr Asn Thr Asp Gly Asn Gly His Gly
        50              55              60

Thr His Val Ala Gly Thr Val Ala Ala Leu Asp Asn Thr Thr Gly Val
65              70              75              80

Leu Gly Val Ala Pro Ser Val Ser Leu Tyr Ala Val Lys Val Leu Asn
            85              90              95

Ser Ser Gly Ser Gly Ser Tyr Ser Gly Ile Val Ser Gly Ile Glu Trp
            100             105             110

Ala Thr Thr Asn Gly Met Asp Val Ile Asn Met Ser Leu Gly Gly Ala
        115             120             125

Ser Gly Ser Thr Ala Met Lys Gln Ala Val Asp Asn Ala Tyr Ala Arg
        130             135             140
```

Gly Val Val Val Val Ala Ala Ala Gly Asn Ser Gly Ser Ser Gly Asn
145                 150                 155                 160

Thr Asn Thr Ile Gly Tyr Pro Ala Lys Tyr Asp Ser Val Ile Ala Val
                165                 170                 175

Gly Ala Val Asp Ser Asn Ser Asn Arg Ala Ser Phe Ser Ser Val Gly
                180                 185                 190

Ala Glu Leu Glu Val Met Ala Pro Gly Ala Gly Val Tyr Ser Thr Tyr
        195                 200                 205

Pro Thr Asn Thr Tyr Ala Thr Leu Asn Gly Thr Ser Met Ala Ser Pro
    210                 215                 220

His Val Ala Gly Ala Ala Ala Leu Ile Leu Ser Lys His Pro Asn Leu
225                 230                 235                 240

Ser Ala Ser Gln Val Arg Asn Arg Leu Ser Ser Thr Ala Thr Tyr Leu
                245                 250                 255

Gly Ser Ser Phe Tyr Tyr Gly Lys Gly Leu Ile Asn Val Glu Ala Ala
                260                 265                 270

Ala Gln

<210> 3
<211> 178
<212> PRT
<213> Thermoascus aurantiacus

<220>
<221> mat_peptide
<222> (1) .. (178)

<400> 3

```
Arg Thr Arg Ile Ser Ser Cys Ser Gly Ser Arg Gln Ser Ala Leu Thr
1               5               10              15

Thr Ala Leu Arg Asn Ala Ala Ser Leu Ala Asn Ala Ala Ala Asp Ala
            20              25              30

Ala Gln Ser Gly Ser Ala Ser Lys Phe Ser Glu Tyr Phe Lys Thr Thr
            35              40              45

Ser Ser Ser Thr Arg Gln Thr Val Ala Ala Arg Leu Arg Ala Val Ala
    50              55              60

Arg Glu Ala Ser Ser Ser Ser Ser Gly Ala Thr Thr Tyr Tyr Cys Asp
65              70              75              80

Asp Pro Tyr Gly Tyr Cys Ser Ser Asn Val Leu Ala Tyr Thr Leu Pro
            85              90              95

Ser Tyr Asn Ile Ile Ala Asn Cys Asp Ile Phe Tyr Thr Tyr Leu Pro
        100             105             110

Ala Leu Thr Ser Thr Cys His Ala Gln Asp Gln Ala Thr Thr Ala Leu
        115             120             125

His Glu Phe Thr His Ala Pro Gly Val Tyr Ser Pro Gly Thr Asp Asp
    130             135             140

Leu Ala Tyr Gly Tyr Gln Ala Ala Met Gly Leu Ser Ser Ser Gln Ala
145             150             155             160

Val Met Asn Ala Asp Thr Tyr Ala Leu Tyr Ala Asn Ala Ile Tyr Leu
            165             170             175

Gly Cys
```